# EUROPEAN PATENT SPECIFICATION

(11) **EP 3 512 598 B1**
(45) Date of publication and mention of the grant of the patent: **04.10.2023**
(21) Application number: 17784005.5
(22) Date of filing: 15.09.2017
(51) Int. Cl.: A61N 2/02

(54) **SUPPORT STRUCTURE FOR A HUMAN BODY FOR MAGNETOTHERAPY**
STÜTZSTRUKTUR FÜR DEN MENSCHLICHEN KÖRPER FÜR MAGNETTHERAPIE
STRUCTURE SUPPORT POUR LE CORPS HUMAIN DESTINÉE À ÊTRE UTILISÉE EN MAGNÉTOTHÉRAPIE

(30) Priority: 15.09.2016 IT 201600093158
(43) Date of publication of application: 24.07.2019
(73) Proprietor: Amel Medical Division S.r.l., 36016 Thiene (VI) (IT)
(72) Inventor: POLI, Luigi, 35010 S. Pietro in Gu (PD) (IT)
(74) Representative: Autuori, Angelo
(86) International application number: PCT/IB2017/055590
(87) International publication number: WO 2018/051279

(56) References cited:
- EP-A1- 0 209 246
- EP-A1- 2 272 561
- EP-A2- 0 850 665
- WO-A1-00/07665
- WO-A1-97/46277
- WO-A1-2013/091585
- WO-A1-2013/117328
- WO-A2-01/15774
- CN-A- 105 749 424
- DE-A1- 4 136 374
- DE-U1- 9 300 499
- GB-A- 2 303 066

## Description

### Technical Field

The present invention finds its application in the technical field of devices for therapeutic and medical use and its particular object is a support structure for the human body designed for use in magnetotherapy treatments.

The invention also relates to a sitting for the human body comprising or integrating thereinside said support structure.

### State of the art

As known, magnetotherapy is a particular therapeutic technique that involves the application of a magnetic or electromagnetic field on one or more areas of the body in order to cure certain pathologies or physical diseases.

Generally, the application of the magnetic or electromagnetic field is carried out locally on the area of the body to be treated by specific applicators whose configuration also varies according to the type of generated field.

For example, in low-frequency magnetotherapy, wherein an electromagnetic field is generated at frequencies close to 100-150Hz, two or four solenoids are electrically powered by a generator and placed in contact or close to the human body part to be treated.

However, such solutions have different drawbacks, mainly because they do not allow a widespread application of the magnetic field, being also uncomfortable to use, with the consequence that it is not possible to have continuity of treatment and prolonged use of magnetotherapy on large parts of the body.

An example of such an application is disclosed in WO00/07665, wherein a magnetotherapy device for the treatment of osteoporosis comprises a planar support designed to be placed on a seat and having a seat plane and a backrest each provided with a solenoid extending in most of the seat and backrest.

WO2013/091585 discloses instead a magnetotherapeutic device for the treatment of cellulite having a support plane for the sitting of a patient wherein a plurality of coils electrically powered is inserted for generating an electromagnetic field.

The coils are powered to generate an electromagnetic field having a value up to 10mT, with fixed frequency values ranging from 1 to 100Hz.

However, it has been shown that these values of the electromagnetic field are not effective as they are too high.

In addition, to obtain such field values, relatively large coils need to be set up, resulting in the creation of localized emission zones, so that it is not possible to have a uniform distribution of the electromagnetic field throughout the support area.

In the case of treatments with high frequency electromagnetic fields, wherein the frequency can reach values up to 5000 Hz, there are known apparatuses designed to emit a diffused magnetic field.

In particular, there are known flexible applicators made up of special fabrics or net fabrics formed by conductive elements, usually copper wires, connected to a field generator so as to have a diffused magnetic field.

These applicators have the advantage of being able to be placed on a support surface for the human body, such as seats, loungers and the like, to allow the user to undergo the treatment in a widespread and comfortable manner.

However, high frequency magnetotherapy has not been as effective as low frequency because of the lower intensity of the electromagnetic field.

In addition, these known devices always require a support structure.

Last but not least, known solution always require the use of fixed frequencies, though adjustable within predetermined ranges before the start of therapy, but in any case not variable during a same therapeutic cycle in order to intervene in an optimal manner against various diseases. An additional kind of magnetotherapy is the stable field one, wherein the magnetic field is generated by permanent magnets.

CN104707253 and CN101396586 describe two seats that integrate thereinto a plurality of permanent magnets.

However, the weakness of the magnetic field generated by permanent magnets makes this therapy ineffective. Moreover, this type of therapy is not yet recognized at the medical level due to a poor supporting bibliography.

### Scope of the invention

The invention is defined by the claims.

An object of the present invention is to overcome the above mentioned drawbacks, providing a support structure for the human body for magnetotherapy that is characterized by high efficiency and relative cheapness.

A particular object is to provide a support structure for the human body for magnetotherapy to allow for a magnetotherapy treatment in a widespread and continuous manner, resulting in particularly comfortable treatment for the user.

Another object is to provide a support structure for the human body for magnetotherapy, which allows the user to carry out the therapy autonomously, even in the case of people with motor impairments such as the elderly or people with particular pathologies.

Yet another object is to provide a support structure for the human body for magnetotherapy, which allows to intervene on several pathologies during the same therapeutic session.

Not least object is to provide a seat that integrates such a support structure for the human body for magnetotherapy, which allows to perform a therapy in an effective, diffused, comfortable manner and in full autonomy for the user.

Such objects, as well as others that will become clearer hereinafter, are achieved by a support structure for human body for magnetotherapy according to claim 1.

The structure so arranged may itself support the body of the user or part thereof or be placed on a further seat or other support for the human body such as a bed, cot, mattress or the like to allow the user to undergo to therapy in a comfortable and continuous way as it will not be necessary to move the emitters along the various parts of the body to be treated.

Emitters of the support structure will orient their respective magnetic fields on one or more areas of the body even at the same time in order to allow therapy to be carried out on one or more areas of the body in a particularly effective and comfortable way, avoiding the risk of misplacement of the emitters and without there is a need for technical support staff.

Advantageous embodiments of the invention are obtained according to the dependent claims.

### Brief disclosure of the drawings

Further features and advantages of the invention will become more apparent in the light of the detailed description of preferred but not limiting embodiments of the structure and of the sitting according to the present invention, illustrated by way of non-limiting example with the aid of the drawing wherein:
**FIG. 1** is a schematic view of a support structure for magnetotherapy;
**FIG. 2** is a schematic view of a support structure for magnetotherapy;
**FIG. 3** is a schematic view of a sitting embedding the support structure.

### Best modes of carrying out the invention

With reference to the accompanying drawings, some embodiments of a support structure for human body that can be used in magnetotherapy for the treatment of various diseases and/or pathologies, such as muscular, articular, bony, vascular pathologies or just to give a benefit to the body even in absence of specific pathologies or diseases.

The structure, generally designated by **1,** essentially comprises at least one panel **2** having a support surface **3** for a part of the human body and preferably enclosing a padding, not visible, whose features may vary depending on the degree of comfort to be obtained and which are not limitative for the present invention.

The panel **2** is also associated with means **4** for generating a magnetic or electromagnetic field having a plurality of emitters **5** that will be integrated within the panel **2** itself, appropriately wound or at least covered by the padding so as not to be uncomfortable for the user, so that their respective magnetic or electromagnetic fields may be oriented directly to the respective areas to be treated.

The field generating means **4** also comprise an electrical power supply unit **6,** external to the panel **2** as in the figures or incorporated thereinto, which will be connected to the emitters **5** for generating a low and/or high frequency electromagnetic field.

In the preferred but not exclusive embodiment of the figures, field generating means **4** will be sized to produce a low frequency electromagnetic field, i.e. frequency which will preferably be less than 130Hz and even more preferably less than 100Hz.

In an exemplary manner, the field strength may be between 2mT and 5mT and preferably close to 3mT, or more preferably close to 2mT, although even slightly higher values may be obtained if allowed by the technical features of the field generating means **4** and of the power supply unit **6,** without any particular theoretical limitations.

In addition, the field generating means **4** may be of adjustable type in order to change the frequency and/or power and/or amperage values according to the needs and their technical features.

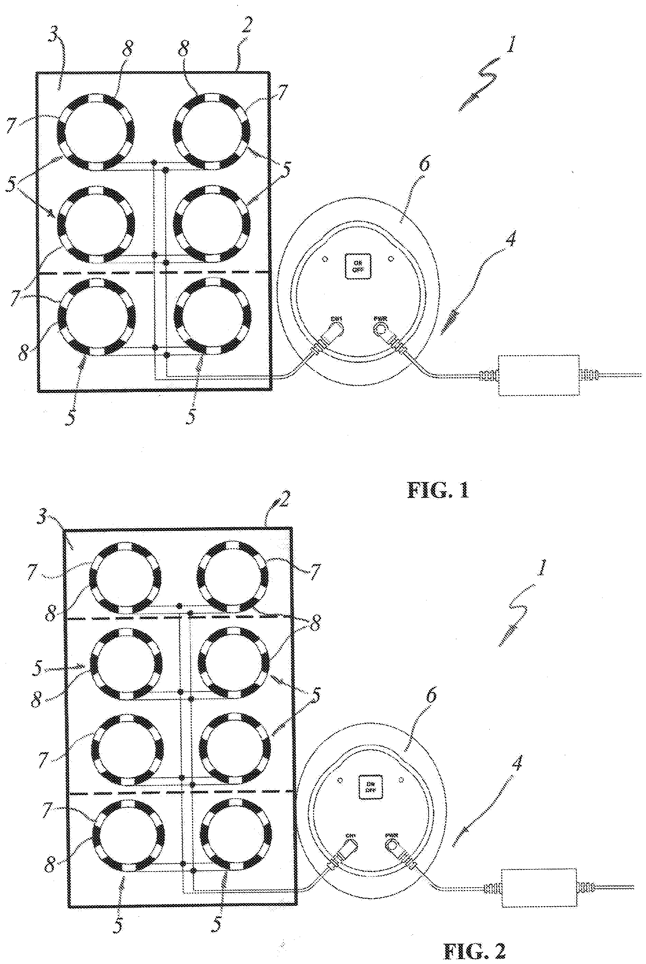

In **Fig. 1** there are six emitters **5** connected in parallel with each other and connected to a current power supply **6** suitable for supplying them with a current having voltage ranging from 5A to 8A at 12VDC fixed voltage.

The emitters **5** are substantially planar and each comprises an annular coil **7** provided with a conductive winding **8,** preferably but not exclusively made of copper, suitable to be connected to the power supply unit **6.**

Suitably, the annular emitters **5** may have an outer diameter between 130mm and 180mm and an inner diameter between 80mm and 120mm although these measurements are purely indicative and may vary according to the size of the panel **2** and of the field intensity to be supplied.

However, such sizes will be preferred as they will allow for a sufficiently large and not excessively localized application area.

According to a purely exemplifying but not exclusive embodiment of the present invention, the emitters **5** can be sized to allow a current to be circulated at 0.8A intensity and generate each a magnetic field between 2mT and 5mT.

**Fig. 2** shows a variant of the previous embodiment wherein there are eight field emitters **5** substantially similar to those described above.

Field generating means **4** may also be configured to power the pairs of emitters **5** selectively so that one or more pairs of emitters may be temporarily excluded depending on the area of the body to undergo to magnetotherapy treatment.

According to a not shown variant, the field generating means **4** of the electromagnetic field may be designed to emit a high frequency field.

In this case, in way of example, the emitters may be defined by a net or fabric in conductive material connected to the electric power supply and integrated inside the panel **2** provided with a padding.

Regardless of the type of generating means, the panel **2** may be a single body or may be formed of two or mutually portions adapted to be reciprocally inclined to allow it to be positioned on a sitting or other support for the human body having reciprocally inclined surfaces.

For example, the panel **2** of **Fig. 1** has two portions **2', 2"** adapted to be reciprocally inclined to be arranged respectively on the backrest and seat of a chair, armchair or other seat.

The panel **2** of **Fig. 2** has instead three portions **2', 2", 2‴** adapted to be reciprocally inclined to be arranged respectively on the headboard, on the backrest and on the seat of a chair, armchair or other sitting.

In this way the panel **2** may be placed on a chair, armchair, bed or other support, adjusting its conformance to the specific shape of the support.

Each of the portions **2', 2", 2‴** of the panel **2** will also be provided with at least one pair of emitters **5** to define respective localized treatment areas.

According to the invention, the field generating means **4** is designed to continuously vary the frequency within a predetermined range, between 10Hz and 130Hz, and possibly also the field intensity.

In this way, through a single cycle of treatment, it is possible to cure various pathologies or diseases because it has been shown that each specific pathology corresponds to an optimal value of the frequency and/or intensity of the magnetic or electromagnetic field.

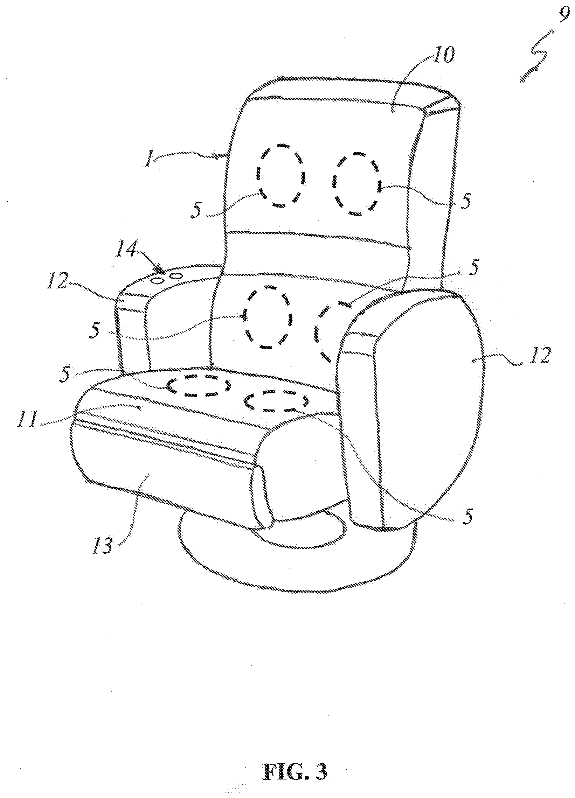

**Fig. 3** shows a magnetotherapy sitting, generally designated by **9,** embedding thereinto a structure **1** according to the invention for generating a low frequency electromagnetic field.

In particular, the structure **1** comprises the backrest **10,** wherein two pairs of emitters **5** are integrated so as to be able to treat the back and lumbar region, and the seating portion **11,** provided with a further pair of emitters **5** for the treatment of the legs.

It is also obvious that there may be a different number of emitters **5,** for example eight as in **Fig. 2****,** depending on the areas to be treated.

In an exemplary manner, further emitters may also be integrated within the lateral armrests **12** and/or in the thigh section **13.**

The power supply unit **6** may be integrated within the same sitting **9** or may be arranged outside. Moreover, the field generating means **4** will also be provided with control means **14** adapted for adjusting the field, the selective power of the emitters, switching on and off the generating means **4.**

The control means **14** may be defined by a push-button station integrated in the same sitting **9,** for example in one of the armrests **12,** or inside an external remote control, not shown.

The control means **14** may also be provided with a timer adapted to stop the treatment after a predetermined maximum time, which may be fixed or adjustable by the user.

According to a not shown variant, the sitting **9** may integrate thereinside means for generating a high frequency electromagnetic field.

According to another variant, also not shown, the sitting **9** may have sensor means for detecting the person. The sensor means may be connected to the control means **13** to enable automatic activation of the generating means **4** upon the detection of the presence of the user on the panel **2** or on the sitting **9** for the automatic starting of the therapy.

According to yet another variant, the panel **2** may comprise a plurality of LED lights connected to the power supply unit **6** to be activated when activating upon the activation of the generating means **4,** signaling their operation.

In addition, the lights will emit a light beam with frequency equal to the frequency of the electromagnetic field, so that the mode of operation is immediately visible.

The lights may be arranged in any position but preferably without any particular limitations. From above it is apparent that the structure and the sitting according to the invention achieve the intended objects.

Although the structure and the sitting have been described with particular reference to the attached figures, the reference numbers used in the description and claims are used to improve the intelligence of the invention and do not constitute any limitation to the claimed scope of protection.

## Claims

1. A support structure for a
human body for magnetotherapy, comprising:
- at least one panel (2) having a supporting surface (3) for a part of the human body;
- means (4) for generating an electromagnetic field having a plurality of emitters (5) integrated into said panel (2);
- an electric supply unit (6) connected to said emitters (5) for generating a low and/or high frequency electromagnetic field;
wherein said field generating means (4) are adapted to produce an electromagnetic field having value ranging from 2mT to 5mT;
wherein said field generating means (4) are adapted to produce an electromagnetic field for magnetotherapy treatments; and
**characterized in that** said field generating means (4) are adapted to vary in a continuous manner the frequency of the field for carrying out a frequency sweep, said frequency being variable within a range of predetermined values comprised between 10Hz and 130Hz.

2. Structure as claimed in claim 1, wherein said frequency is variable within a range of values lower than 100Hz.

3. Structure as claimed in any preceding claim, wherein said emitters (5) are substantially flat and each comprise an annular coil (7) provided with a conducing winding (8) adapted to be connected to said electric supply unit (6).

4. Structure as claimed in claim 3, wherein said annular coils (7) have an outer diameter between 130mm and 180mm and an inner diameter between 80mm and 120mm.

5. Structure as claimed in any preceding claim, wherein said emitters (5) are connected to said power supply unit (6) to be powered with a current having intensity between 5A and 8A with 12VDC fixed voltage.

6. Structure as claimed in any preceding claim, wherein said emitters (5) are sized to allow the circulation of a current having intensity of 0,8A.

7. Structure as claimed in any claim from 3 to 6, wherein said emitters (5) are connected together in parallel.

8. Structure as claimed in any preceding claim, wherein said generating means (4) are adapted to produce a low-frequency electromagnetic field and to feed said emitters (5) in a selective manner.

9. Structure as claimed in any preceding claim, wherein said panel (2) comprises an internal padding enveloping said emitters (5).

10. Structure as claimed in any preceding claim, wherein said panel (2) comprises two or more mutually inclinable portions (2', 2", 2‴) to be placed on a sitting or other support for the human body having bearing surfaces mutually inclined, each of said portions (2', 2", 2‴) being provided with at least one pair of said emitters (5) to define respective localized treatment areas.

11. Structure as claimed in any preceding claim, wherein said panel (2) comprises a plurality of LED lights connected to said electrical power supply unit (6) for signaling the operation of said field generating means (4), said lights being adapted to emit a beam of light at a frequency equal to the frequency of said electromagnetic field.

12. A seat for magnetotherapy (9) integrating thereinside a structure (1) according to one or more of the preceding claims.

13. Seat as claimed in claim 12, wherein said structure (1) comprises at least one backrest (10) and a seat portion (11) each having at least one pair of emitters (5) integrated thereinside, said electric supply unit (6) being integrated in said structure (1) and being optionally provided with control means (13) for controlling the power supplying of said field generating means (4).

## Patentansprüche

1. Eine Trägerstruktur für einen menschlichen Körper für die Magnettherapie, umfassend:
- mindestens eine Platte (2) mit einer Auflagefläche (3) für einen Teil des menschlichen Körpers;
- Mittel (4) zur Erzeugung eines elektromagnetischen Feldes mit einer Vielzahl von Emittern (5), die in die Platte (2) integriert sind;
- eine elektrische Versorgungseinheit (6), die mit den Emittern (5) verbunden ist, um ein nieder- und/oder hochfrequentes elektromagnetisches Feld zu erzeugen;
wobei die das Feld erzeugenden Mittel (4) so ausgelegt sind, dass sie ein elektromagnetisches Feld mit einem Wert im Bereich von 2mT bis 5mT erzeugen; wobei die das Feld erzeugenden Mittel (4) so ausgelegt sind, dass sie ein elektromagnetisches Feld für Magnettherapiebehandlungen erzeugen; und **dadurch gekennzeichnet, dass** die das Feld erzeugenden Mittel (4) dazu ausgebildet sind, die Frequenz des Feldes kontinuierlich zu verändern, um einen Frequenzdurchlauf auszuführen, wobei die Frequenz innerhalb eines Bereichs von vorbestimmten Werten variabel ist, die von einem Bereich zwischen 10Hz und 130Hz umfasst sind.

2. Struktur nach Anspruch 1, wobei die Frequenz variabel ist innerhalb eines Wertebereichs von weniger als 100 Hz.

3. Struktur nach einem der vorhergehenden Ansprüche, wobei die Emitter (5) im Wesentlichen flach sind und jeweils eine ringförmige Spule (7) aufweisen, die mit einer leitenden Wicklung versehen ist (8), die zur Verbindung mit der elektrischen Versorgungseinheit (6) ausgebildet ist.

4. Struktur nach Anspruch 3, wobei die ringförmigen Spulen (7) einen Außendurchmesser zwischen 130 mm und 180 mm und einen Innendurchmesser zwischen 80 mm und 120 mm aufweisen.

5. Struktur nach einem der vorhergehenden Ansprüche, wobei die Emitter (5) mit der Stromversorgungseinheit (6) verbunden sind, um mit einem Strom mit einer Stärke zwischen 5A und 8A bei einer festen Spannung von 12VDC versorgt zu werden.

6. Struktur nach einem der vorhergehenden Ansprüche, wobei die Emitter (5) so bemessen sind, dass ein Strom mit einer Stärke von 0,8 A fließen kann.

7. Struktur nach einem der Ansprüche 3 bis 6, wobei die Emitter (5) parallel geschaltet sind.

8. Struktur nach einem der vorhergehenden Ansprüche, wobei die das Feld erzeugenden Mittel (4) so ausgebildet sind, dass sie ein niederfrequentes elektromagnetisches Feld erzeugen und die Emitter (5) selektiv speisen.

9. Struktur nach einem der vorangehenden Ansprüche, wobei die Platte (2) eine innere Polsterung aufweist, die die Emitter (5) umschließt.

10. Struktur nach einem der vorangehenden Ansprüche, wobei die Platte (2) zwei oder mehr gegeneinander neigbare Abschnitte (2', 2", 2‴) umfasst, die auf einer Sitzfläche oder einer anderen Stütze für den menschlichen Körper mit gegeneinander geneigten Auflageflächen zu platzieren sind, wobei jeder der Abschnitte (2', 2", 2‴) mit mindestens einem Paar der Emitter (5) versehen ist, um die jeweiligen lokalisierten Behandlungsbereiche zu definieren.

11. Struktur nach einem der vorhergehenden Ansprüche, wobei die Platte (2) eine Vielzahl von LED-Leuchten umfasst, die mit der elektrischen Stromversorgungseinheit (6) verbunden sind, um den Betrieb der das Feld erzeugenden Mitte (4) zu signalisieren, wobei die Leuchten zur Erzeugung eines Lichtstrahls mit einer Frequenz ausgebildet sind, die der Frequenz des elektromagnetischen Feldes entspricht.

12. Sitz für die Magnettherapie (9), in welchen eine Struktur (1) gemäß nach einem oder mehreren der vorhergehenden Ansprüche integriert ist.

13. Sitz nach Anspruch 12, wobei die Struktur (1) mindestens eine Rückenlehne (10) und einen Sitzteil (11) umfasst, in die jeweils mindestens ein Paar von Emittern (5) integriert ist, wobei die elektrische Versorgungseinheit (6) in die Struktur (1) integriert ist und optional mit Steuermitteln (13) zur Steuerung der Energieversorgung der das Feld erzeugenden Mitte (4) versehen ist.

## Revendications

1. Structure de support destinée à un corps humain pour une magnétothérapie, comprenant :
- au moins un panneau (2) doté d'une surface de support (3) pour une partie du corps humain ;
- des moyens (4) de génération d'un champ électromagnétique ayant une pluralité d'émetteurs (5) intégrés dans ledit panneau (2) ;
- une unité d'alimentation électrique (6) connectée auxdits émetteurs (5) pour générer un champ électromagnétique à fréquence basse et/ou élevée ;
lesdits moyens de génération de champ (4) étant adaptés pour produire un champ électromagnétique ayant une valeur allant de 2mT à 5mT ;
lesdits moyens de génération de champ (4) étant adaptés pour produire un champ électromagnétique pour des traitements de magnétothérapie ; et
**caractérisé en ce que** lesdits moyens de génération de champ (4) sont adaptés pour modifier de manière continue la fréquence du champ pour mettre en oeuvre un balayage de fréquence, ladite fréquence étant variable à l'intérieur d'une plage de valeurs prédéterminées comprises entre 10Hz et 130Hz.

2. Structure selon la revendication 1, dans laquelle ladite fréquence est variable à l'intérieur d'une plage de valeurs inférieures à 100Hz.

3. Structure selon l'une quelconque des revendications précédentes, dans laquelle lesdits émetteurs (5) sont sensiblement plats et chacun comprend une bobine annulaire (7) pourvue d'un enroulement conducteur (8) adapté pour être connecté à ladite unité d'alimentation électrique (6).

4. Structure selon la revendication 3, dans laquelle lesdites bobines annulaires (7) ont un diamètre externe entre 130mm et 180mm et un diamètre interne entre 80mm et 120mm.

5. Structure selon l'une quelconque des revendications précédentes, dans laquelle lesdits émetteurs (5) sont connectés à ladite unité d'alimentation électrique (6) pour être alimentés avec un courant ayant une intensité entre 5A et 8A et une tension fixe de 12VDC.

6. Structure selon l'une quelconque des revendications précédentes, dans laquelle lesdits émetteurs (5) sont dimensionnés pour permettre la circulation d'un courant ayant une intensité de 0,8A.

7. Structure selon l'une quelconque des revendications 3 à 6, dans laquelle lesdits émetteurs (5) sont connectés ensemble en parallèle.

8. Structure selon l'une quelconque des revendications précédentes, dans laquelle lesdits moyens de génération (4) sont adaptés pour produire un champ électromagnétique à fréquence basse et pour alimenter lesdits émetteurs (5) de manière sélective.

9. Structure selon l'une quelconque des revendications précédentes, dans laquelle ledit panneau (2) comprend un rembourrage interne enveloppant lesdits émetteurs (5).

10. Structure selon l'une quelconque des revendications précédentes, dans laquelle ledit panneau (2) comprend au moins deux parties mutuellement inclinables (2', 2", 2‴) destinées à être placées sur un siège ou un autre support pour le corps humain comprenant des surfaces d'appui mutuellement inclinées, chacune desdites parties (2', 2", 2‴) comprenant au moins une paire desdits émetteurs (5) pour définir des zones de traitement localisées respectives.

11. Structure selon l'une quelconque des revendications précédentes, dans laquelle ledit panneau (2) comprend une pluralité de lumières DEL connectées à ladite unité d'alimentation électrique (6) pour signaler le fonctionnement desdits moyens de génération de champ (4), lesdites lumières étant adaptées pour émettre un faisceau de lumière à une fréquence égale à la fréquence dudit champ électromagnétique.

12. Siège destiné à une magnétothérapie (9) intégrant à l'intérieur de celui-ci une structure (1) selon une ou plusieurs des revendications précédentes.

13. Siège selon la revendication 12, dans lequel ladite structure (1) comprend au moins un dossier (10) et une partie de siège (11), chacun ayant au moins une paire d'émetteurs (5) intégrée à l'intérieur de celui-ci, ladite unité d'alimentation électrique (6) étant intégrée dans ladite structure (1) et comprenant éventuellement des moyens de contrôle (13) pour contrôler l'alimentation électrique desdits moyens de génération de champ (4).
